# EUROPEAN PATENT SPECIFICATION

(11) **EP 4 014 024 B1**
(45) Date of publication and mention of the grant of the patent: **01.07.2026**
(21) Application number: 20757232.2
(22) Date of filing: 04.08.2020
(51) Int. Cl.: G01N 21/25, G01N 21/552, G01N 21/64, B01L 3/00, B01L 9/00

(54) **ANALYSING SYSTEM FOR MULTI-WELL SAMPLE CARRIERS**
ANAYSESYSTEM FÜR MULTI-WELL PROBENTRÄGER
SYSTÈME D'ANALYSE POUR LES SUPPORTS D'ÉCHANTILLONS À PUITS MULTIPLES

(30) Priority: 13.08.2019 GB 201911553
(43) Date of publication of application: 22.06.2022
(73) Proprietor: Causeway Sensors Limited, Belfast, County Antrim BT7 1NF (GB)
(72) Inventor: MURPHY, Antony, Belfast County Down BT8 6WP (GB); HILL, Breandan, County Antrim BT36 7JR (GB); POLLARD, Robert, County Down BT20 5HX (GB)
(74) Representative: FRKelly
(86) International application number: PCT/EP2020/071936
(87) International publication number: WO 2021/028275

(56) References cited:
- US-A1- 2003 059 855
- US-A1- 2008 014 632
- US-A1- 2017 370 836
- US-A1- 2018 361 379
- US-A1- 2019 120 762

## Description

### Field of the Invention

The present invention relates to systems and apparatus for analyzing samples, particularly comprising chemical or biological material, held in multi-well plates

### Background to the Invention

Multi-well plates, which are sometimes known as microplates, microwell plates or microtiter plates, are commonly used to hold chemical or biological samples for the purpose of analytical research or diagnostic testing. Analysis of the samples is performed by a plate reader which detects biological, chemical or physical characteristics or events relating to the samples. Conventional plate readers have a light source that illuminates the contents of the wells from above, and an optical detector for receiving light reflected from or emitted by the samples. Known plate readers are relatively expensive and can have relatively insensitive detection capabilities. Also, known plate readers are restricted in the number of wells that can be analysed at a time. US 2008/014632 A1 discloses a multi-well plate reader and multi-well plates wherein the multiple wells are read via dual fibre probes though a transparent bottom of the respective wells. US 2019/120762 A1 discloses a sensor having a transparent body with a nano-structured surface that is coupled to the fluid container such that the nano-structured surface is exposed to the sample chamber and a corresponding reader.

It would be desirable to mitigate the problems outlined above.

### Summary of the Invention

A first aspect of the invention provides an analyzing system according to claim 2 comprising: a sample carrier comprising
a plurality of sample wells, each well having a transparent bottom, the bottom having an obverse face located within the well and a reverse face external to the well, the obverse face having a nanostructured surface comprising a plurality of nanostructures; an reader device comprising a station for receiving the sample carrier, the station comprising a plurality of light guiding units arranged so that a respective light guiding unit is aligned with a respective well when the sample carrier is received by the station, wherein each light guiding unit is configured to direct a beam of excitation radiation to the bottom of the respective well, and to direct a beam of reflected radiation from the bottom of the respective well.

A second aspect of the invention provides a reader device according to claim 1 for an analyzing system, the reader device comprising a station for receiving a multi-well sample carrier, the station comprising a plurality of light guiding units arranged so that a respective light guiding unit is aligned with a respective well of the sample carrier when the sample carrier is received by the station, wherein each light guiding unit is configured to direct a beam of excitation radiation to the bottom of the respective well, and to direct a beam of reflected radiation from the bottom of the respective well.

A third aspect of the disclosure provides a sample carrier for an analyzing system, the sample carrier comprising a plurality of sample wells, each well having a transparent bottom, the bottom having an obverse face located within the well and a reverse face external to the well, the obverse face having a nanostructured surface comprising a plurality of nanostructures.

Preferably, the sample carrier is removably locatable on the station.

The wells are preferably arranged in an array, and said light guiding units are arranged in an array corresponding to the array in which the wells are arranged. The preferred sample carrier comprises a base, the respective well bottoms, in particular the respective reverse face of the well bottoms, being located at said base and preferably being coplanar, or substantially coplanar, with one another. In preferred embodiments, the sample carrier comprises a multi-well plate.

Each light guiding unit comprises an optical port, the light guiding units being arranged so that a respective optical port is aligned with a respective well when the sample carrier is received by the station. Said station comprises a support structure for receiving said sample carrier, ir particular the base of the sample carrier, said optical ports being included in said support structure, preferably arranged in an array, and being exposed by the support structure for coupling with a respective well. The support structure typically comprises a support surface, which is preferably flat or substantially flat, for receiving the base of said sample carrier, wherein said optical ports are incorporated into said support surface, preferably arranged in an array, and being exposed by the support surface for coupling with a respective well.

The well bottom, in particular the reverse face of the well bottom, preferably covers the respective optical port when the sample carrier is received by the station. Each optical port comprises a mouth, preferably an open mouth, and wherein the well bottom, in particular the reverse face of the well bottom, covers the mouth of the respective optical port when the sample carrier is received by the station. In preferred embodiments, the respective well engages with the respective optical port, typically with the mouth of the respective optical port, when the sample carrier is received by the station.

According to the invention, each optical port comprises a cavity, wherein each light guiding unit is configured to direct the beam of excitation radiation to the bottom of the respective well through the cavity, and to direct the beam of reflected radiation from the bottom of the respective well through the cavity. The cavity is located below said support surface.

Each light guiding unit is connected to, or connectable to, an excitation source for generating said beam of excitation radiation, said light guiding unit comprising light guiding means, optionally comprising at least one optical fibre, for directing said beam of excitation radiation to the bottom of the respective well. Said light guiding means are arranged to direct said beam of excitation light from said excitation source to said cavity. Typically, each light guiding unit is connected to, or connectable to, a optical detector, said light guiding unit comprising light guiding means, optionally comprising at least one optical fibre, for directing said reflected beam to said detector. Preferably, said light guiding means is arranged to direct said reflected beam light from the bottom of the respective well through said cavity to said detector.

In preferred embodiments, said nanostructures are elongate, having a respective longitudinal axis that is disposed substantially perpendicularly to the obverse face, and wherein, preferably, the nanostructures are spaced apart from one another by a distance less than the wavelength of the excitation radiation to cause, in use, plasmonic oscillations in a direction that is normal to said obverse face.

In preferred embodiments, the system further comprises an excitation source for generating the beam of excitation radiation for each light guiding unit; an optical detector for detecting the beam of reflected radiation from each light guiding unit; and a controller for controlling operation of the excitation source. The controller is preferably configured to control the excitation source to cause a respective beam of excitation radiation to be directed to the bottom of the respective well by any one of the light guiding units individually, or by any two or more of the light guiding units simultaneously, or by all of the light guiding units simultaneously. The preferred system includes analyzing means, optionally implemented by said controller, configured to analyze one or more output signal from said optical detector. The analyzing means may be configured to analyze data from said at least one output signal in respect of any one of the wells individually, or in respect of any two or more of the wells in combination, or in respect of all of the wells individually or in combination.

Typically, the obverse and reverse faces of the well bottom are substantially parallel with one another. The well bottom may be planar, or cuboid, in shape, said obverse and reverse faces being oppositely disposed on the planar, or cuboid, bottom.

Preferably, each light guiding unit includes a region, preferably the cavity, comprising a medium through which said incident radiation beam travels, in use, to said reverse face of the well bottom, wherein the refractive index of said medium is less than the refractive index of the material from which said bottom is made.

In preferred embodiments, said beam of incident radiation comprises p-polarised radiation. The incident radiation preferably has a wavelength between approximately 300 nm to 1500 nm. The incident radiation is preferably light.

In preferred embodiments, the system is configured to direct said beam of incident polarised electromagnetic radiation onto the reverse face of each well bottom through a first medium with a first refractive index, said bottom being formed from a material with a second refractive index different from said first refractive index, said beam being directed to impinge upon said reverse face substantially at the Brewster angle corresponding to said first and second refractive indicies. Typically, said first medium is air and said second medium is glass, said angle being approximately 57º. The preferred system is configured to direct said beam of incident polarised electromagnetic radiation onto the reverse face of the well bottom such that, upon travelling though said bottom, said incident beam impinges upon said nanostructured surface at an angle less than that required for attenuated total reflection (ATR).

In preferred embodiments, the nanostructures are substantially parallel with each other. The nanostructures may be spaced apart from one another, or at least some and optionally all of the nanostructures are contiguous with the, or each, adjacent nanostructure. The nanostructures may be arranged in a one or two dimensional array. The preferred aspect ratio of the length to the width of each nanostructure is greater than 1. Preferably, at least some and preferably all of the nanostructures are nanoparticles, having three dimensions on the nanoscale. The nanostructures are typically formed from an electrically conductive material, typically a metallic material. The nanostructures may be formed in an electrically conductive layer provided at the obverse face of the well bottom.

Each light guiding unit typically includes light guiding means, such as one or more optical fibre or other light guide, configured to direct the incident radiation beam to, and the reflected radiation beam from, the reverse face of the well bottom. Each light guide unit includes an excitation channel for directing the incident radiation towards the reverse face of the well bottom. The excitation channel may contain one or more optical fibre. Each light guiding unit includes a detection channel for directing the reflected radiation beam from the reverse face of the well bottom towards the detector. The detection channel may contain one or more optical fibre.

Optionally, at least a first region of the nanostructured surface, and in particular the nanostructures on the nanostructured surface, is functionalised with a first member of a primary binding couple having an affinity for a second member of the primary binding couple which is functionalised upon at least some of the nanoentities; and/or wherein the nanoentities are further functionalised with a first member of a secondary binding couple having an affinity for a second member of the secondary binding couple which comprises at least one of the analytes contained within the sample; and/or wherein the analyte is functionalised with the second member of the secondary binding couple. Optionally, at least a second region of the nanostructures, and in particular the nanostructures on the nanostructured surface, is functionalised with a first member of a tertiary binding couple having an affinity for a second member of the tertiary binding couple which is coated upon some of the nanoentities; and/or wherein the nanoentities which are functionalised with the second member of the tertiary binding couple are further functionalised with a first member of a quaternary binding couple which has an affinity for a second member of the quaternary binding couple which comprises at least one of the analytes contained within the sample; and/or wherein the analyte is functionalised with the second member of the quaternary binding couple.

The binding couples may comprise receptor-ligand binding couples. The first member of the primary and/or tertiary binding couples may comprise an antibody and the second member of the primary binding couple comprises a complimentary antigen. The first member of the secondary and/or quaternary binding couple may comprise an antibody and the second member may comprise a complimentary antigen. The nanostructures and/or nanoentities and/or analyte may be functionalised by being coated with the respective binding member or members. Optionally, each of a plurality of regions of the nanostructured surface are connected to a respective electrical terminal; and/or wherein respective different electrical bias may be applied to each of the regions by respective power supplies coupled to the electrical terminals.

Optionally, the respective nanostructures of each of a plurality of regions of the nanostructured surface are configured to resonate when illuminated by radiation at respective different wavelengths; and/or wherein the respective nanostructures of each region are configured to resonate at a respective wavelength that corresponds to a respective wavelength of the radiation produced by said excitation source; and/or wherein said excitation source is operable to produce radiation at more than one wavelength, and wherein said nanostructured surface includes at least one of said nanostructured region for each of said wavelengths in which the respective nanostructures are configured to resonate when illuminated by the radiation at the respective wavelength.

Preferred systems embodying the invention allow for highly sensitive quantification and characterisation of biological interactions in real time, with minimal intervention in the form of additional labelling steps. This label-free response allows monitoring of process/manufacturing steps with resulting improvements in yields, quality and production times. In addition, the scalability of the system and the ability to take many readings in parallel improves throughput greatly and allows commonly used formats (e.g. 96, 384-well plates) to be addressed in ways not previously possible.

Advantageously, the ability to carry out these measurements in parallel means that each well can be treated independently with respect to time, which give several unique advantages in applications, for example in cases where reagents degrade or denature; or where reactions occur very quickly; or where: immobilisation or incubation times are very long and are therefore are desirable not to be repeated; or where a laboratory may lack the ability to add reagents to all wells simultaneously and therefore reactions may start at different times; or where measurements of multiple types are required simultaneously e.g. SPR and (metal-enhanced) fluorescence.

In order to produce high sensitivity, preferred embodiments may promote plasmonic disruption with nanoentities. Optionally, the plasmonic disruption may be coupled with a fluorescent or fluorescently tagged nanoentity, allowing the system to obtain simultaneous information from plasmonic resonance shifts and enhanced fluorescent measurements.

Further advantageous aspects of the invention will be apparent to those ordinarily skilled in the art upon review of the following description of a specific embodiment and with reference to the accompanying drawings.

### Brief Description of the Drawings

An embodiment of the invention is now described by way of example and with reference to the accompanying drawings in which:
Figure 1 is a perspective view of an analysing system embodying one aspect of the invention, the system being shown with a multi-well sample carrier in a removed state;
Figure 2 is a perspective view of the system of Figure 1 in which the sample carrier is shown in an installed state;
Figure 3 is a cut-away perspective view of the multi-well sample carrier;
Figure 3A is a detail illustration of a nanostructured surface provided at the bottom of each well of the multi-well sample carrier;
Figure 4 is a schematic illustration of part of the analysing system of Figure 1 with the sample carrier in the installed state;

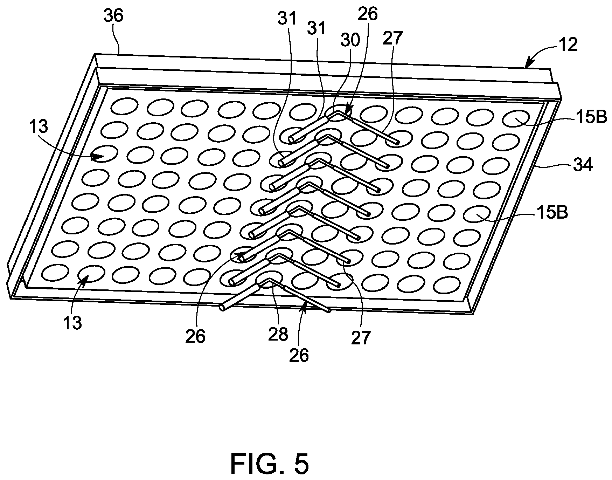

Figure 5 is a perspective view of a sample carrier receiving part of the system of Figure 1, shown from the underside, and showing only some light guiding units for illustrative purposes;

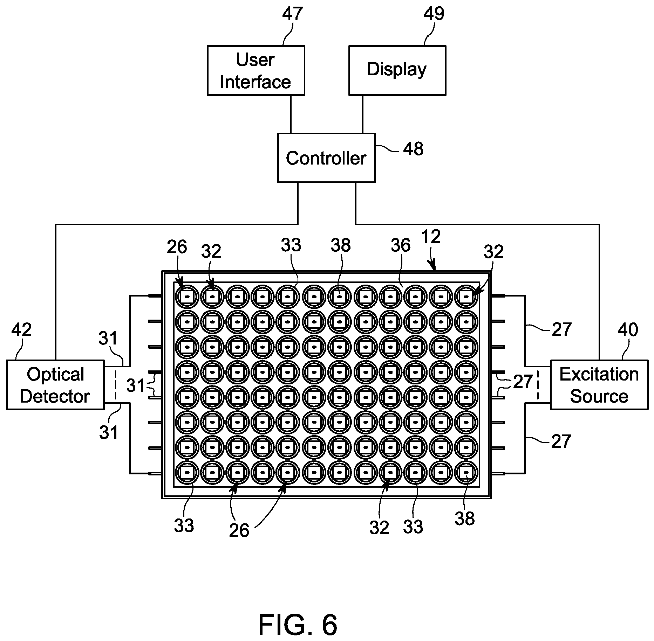

Figure 6 is a schematic view of the system of Figure 1;
Figure 7 is a detail of the nanostructured surface of Figure 3A in particular showing a plurality of nanoentities adjacent thereto;
Figure 8 is a schematic view of the analysing system wherein a region of the nanostructured surface is coated in a first member of a first binding couple;
Figure 8A is a schematic view of a nanoentity, in this case a nanosphere, which is coated in a second member of the first binding couple and a first member of a second binding couple;
Figure 8B is a schematic view of an analyte, in this case a pathogen, which comprises the second member of the second binding couple;
Figure 8C is a schematic view showing the binding of the analyte and nanoentity, in particular showing a nanoentity and analyte complex formed by the binding of the first and second members of the second binding couple.
Figure 8D is a schematic view of the nanostructured surface showing the analyte immobilised on the nanostructured surface by the binding of analyte to the nanoentity via the second binding couple and the binding of the nanoentity to the nanostructured surface by the first binding couple;
Figure 9 is a schematic diagram of an alternative embodiment showing an excitation apparatus directing a beam of polarised radiation at the plasmonic nanostructured surface and a corresponding detection apparatus, in particular showing first and second regions of the nanostructured surface with each region being coated in a different first member of a respective binding couple.
Figure 9A is a schematic diagram of two nanoentities with the nanoentities being coated in respective first and second members of first and second binding couples such that the nanoentities are operable to immobilise different analytes upon the first and second regions of the nanostructured surface as shown in figure 9;
Figure 9B is a schematic diagram showing two different analytes with each analyte comprising different binding members for binding to the differently functionalised nanoentities of figure 9A; and
Figure 9C is a schematic diagram showing the binding of the different analytes to the two nanoentities by the interaction of the different binding members of the binding couples.

### Detailed Description of the Drawings

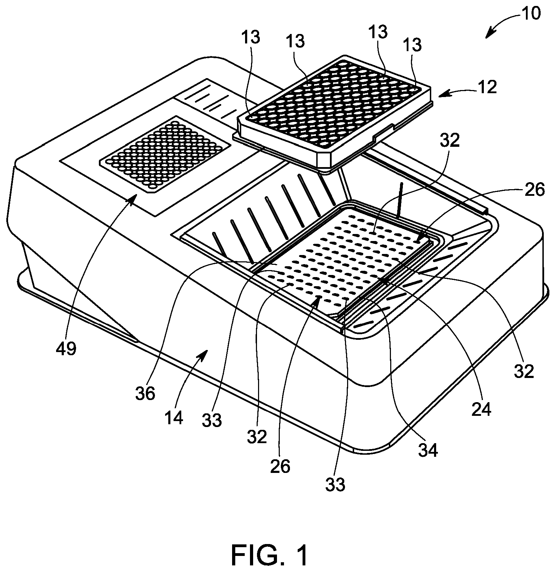

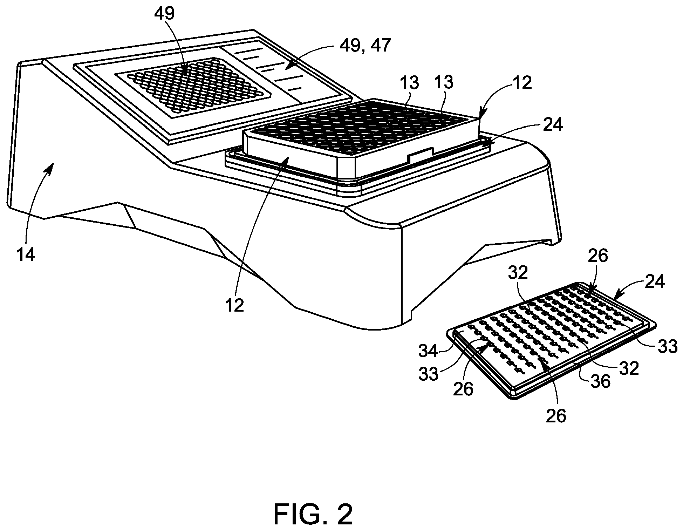

Referring now to the drawings there is shown, generally indicated as 10, an analysing system embodying one aspect of the invention. The system 10 comprises a sample carrier 12 and a reader device 14. Advantageously, the sample carrier 12 is removable from the reader device 14. Figure 1 shows the sample carrier 12 in a removed state in which it may for example be filled with samples or may be washed, and Figure 2 shows the sample carrier 12 in an installed state in which the system 10 is able to perform analysis of the contents of the sample carrier 12 as is described on more detail hereinafter.

The sample carrier 12 comprises a plurality of sample wells 13, typically arranged in an array, usually a two dimensional or rectangular array. In preferred embodiments, the sample carrier 12 is a multi-well plate, for example of a type known as a microwell plate, microplate or microtiter plate. Each well 13 has a closed bottom 15 that is optically transparent, and typically has an open top or mouth 21. In use, each well 13 contains a sample (not shown) to be analyzed. Typically the sample in each well 13 comprises chemical or biological material, usually in a liquid. The wells 13 may contain the same sample material or different sample material as required by the application. The well bottoms 15, in particular the respective reverse face of the well bottoms, are located at the base of the sample carrier 12, and are preferably being coplanar, or substantially coplanar, with one another. The base of the sample carrier 12 may be flat or substantially flat.

The sample carrier 12 may have, for example, 6, 12, 24, 48, 96, 384 or 356 wells 13 arranged in a rectangular array, although may more generally have any number of wells 13. Typical volumes for the wells 13 range from the order of nanolitres to the order of millilitres. Usually, the wells 13 are of the same size.

The bottom 15 has an obverse face 15A located within the well 13 and a reverse face 15B external to the well 13. The obverse face 15A has a nanostructured surface comprising a plurality of nanostructures 16. The nanostructured surface, and therefore the nanostructures 16, are exposed to the inside of the well 13 and therefore to any sample contained in the well 13 during use. The nanostructured surface is configured so that the bottom 15 of each well 13 acts as a nanostructured plasmonic sensor. In particular, when the nanostructured surface is suitably illuminated, plasmonic resonance occurs resulting in the generations of plasmons on the nanostructured surface. The nanostructured surface may therefore be described as a plasmonically active surface. The plasmonic resonance may be of a type known as surface plasmon resonance (SPR) in which surface plasmons are generated in the nanostructured surface. Interactions between the plasmons and the sample in the well 13 affects light reflected from the nanostructured surface and these affects can be detected by an optical detector in order to analyse the sample.

A nanostructured surface is a surface on which there is formed a plurality of nanostructures. A nanostructure is a structure that has at least one dimension on the nanoscale. For the purposes of the present invention, nanoscale means between 0.1 nm and 1000 nm, more typically between 1 nm and 100 nm. A nanostructure may have only one dimension on the nanoscale, or two dimensions on the nanoscale, or three dimensions on the nanoscale. Nanostructures having three dimensions on the nanoscale are referred to as nanoparticles.

The nanostructured surface provided on the obverse face 15A may take any form that allows it to act as a plasmonic sensor. In some embodiments, the nanostructured surface may comprise any known SPR sensor surface. In some embodiments, the nanostructures have their longitudinal axis disposed parallel with the face 15A, but in preferred embodiments, the nanostructures have their longitudinal axis disposed perpendicularly to the face 15A.

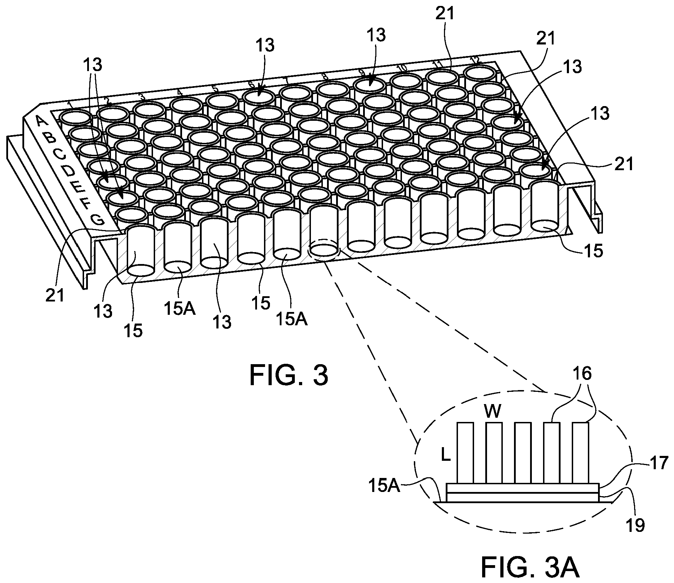

Figure 3Ashows a detail view of the preferred nanostructured surface of the obverse face 15A. The nanostructured surface comprises a plurality of nanostructures 16 that are preferably elongate, having a respective longitudinal axis that is disposed substantially perpendicularly to the obverse face 15A. The nanostructures 16 are therefore substantially parallel with each other. In preferred embodiments, the nanostructures 16 are spaced apart from one another, e.g. each nanostructure 16 is spaced apart from the, or each, adjacent nanostructure 16. Alternatively, at least some and optionally all of the nanostructures 16 are contiguous with the, or each, adjacent nanostructure 16. The nanostructures 16 may be arranged in a one or two dimensional array, preferably being aligned with each other along the, or each, dimension of the array. Advantageously, the aspect ratio of the length L to the width W of each nanostructure 16 is greater than 1. The third dimension (not illustrated) of the nanostructures 16 may be of any desired size depending on the application, for example it may be similar to the width W or the length L, or may be unlimited, e.g. the nanostructures may form a grating. In preferred embodiments, at least some and preferably all of the nanostructures 16 are nanoparticles, having three dimensions on the nanoscale.

For example, some or all of the nanostructures 16 may comprise a wire or a tube, in particular a nanowire or nanotube, which may take any suitable shape for example substantially cylindrical or substantially conical. The nanostructures 16 may be solid or hollow.

The nanostructures 16 are typically formed from an electrically conductive material, typically a metallic material, for example, any of silver, gold, aluminium, platinum, copper or any noble metal or any combinations of the aforesaid.

In typical embodiments, the width W of the nanostructures 16 is approximately 2 nm to approximately 500 nm, usually approximately 10 nm to approximately 100 nm, and the length L of the nanostructures 16 is approximately 10 nm to approximately 2000 nm, usually approximately 50 nm to approximately 500 nm. For example, the nanostructures 16 may have a width of approximately 20 nm and a length of approximately 500 nm. It is preferred that the nanostructures 16 are substantially uniform in width and/or height although this need not necessarily be the case.

Typically, the spacing between adjacent nanostructures 16 is approximately 2 nm to approximately 1500 nm, usually approximately 20 nm to approximately 500 nm. In preferred embodiments, the nanostructures 16 are spaced apart from one another by a distance less than the wavelength of the excitation light used to cause plasmonic oscillations, as is described in further detail below. The nanoparticle to nanoparticle separation may be periodic, at a scale of approximately 20 nm to approximately 1500 nm. The nanoparticle to nanoparticle separation may be quasi-periodic, at a scale of approximately 20 nm to approximately 1500 nm. Typically, the surface 15A includes in the order of one billion nanoparticles 16.

The well bottom 15 provides mechanical support for the nanostructures 16. The bottom 15 may be made of any convenient material, preferably a dielectric material, for example glass, crystal or plastics. Typically, the bottom 15 is substantially planar in shape, having spaced apart, oppositely disposed obverse and reverse faces 15A, 15B that are preferably parallel with one another. By way of example, the bottom 15 may be between approximately 0.3 to approximately 2 mm thick (i.e. between faces 15A, 15B. The bottom 15 is made from material that is transparent to the electromagnetic radiation (usually light radiation) that is used to illuminate the nanostructured surface, as is described in more detail below. In the case where the illuminating radiation is light, the bottom 15 may conveniently be formed from glass.

In typical embodiments, the nanostructures 16 are formed on a layer 17 of electrically conductive material, typically a metallic layer, provided at the obverse face 4 of the body 12, i.e. as part of a nanostructured metallic layer on the obverse face 15A. Any conventional fabrication technique that is suitable for forming a nanostructured metallic layer may be used for this purpose. For example, the nanostructures may be formed by electrodeposition, optionally in pores formed in a layer of insulating material, e.g. aluminium oxide. Typically, an adhesive layer 19 is provided between the obverse face 15A and the metallic layer 17. Any conventional adhesive layer material may be used, e.g. titanium or tantalum, and is typically formed by physical vapour deposition. In alternative embodiments, the layers 17, 19 may be omitted and the nanostructures may be provided on the face 15A by any other means, e.g.by dispersion of a liquid with a dispersion of nanostructures and subsequent evaporation of the liquid.

The plasmonic nanostructures 16 may be produced using nano-injection moulding. The wells 13 and plasmonic surfaces 15A may be bonded using laser welding or ultrasonic welding. Each well 13 may be chemically treated to form a biologically active monolayer. The monolayer may be configured to reduce non-specific binding of other biological molecules. Each well 13 may comprise multiple discrete spots of biological material.

In use, the nanostructures 16 exhibit localised surface plasmon resonances whose wavelengths are dependent on the size and shape of the nanostructure. The resonance wavelength condition is also sensitive to the surrounding refractive index. Interactions of biological samples at the nanostructure surface causes a spectral resonance shift, which can be detected and analysed as is described in more detail hereinafter.

The reader device 14 comprises a station 24 for receiving the sample carrier 12. The station 24 comprises a plurality of light guiding units 26 arranged so that a respective light guiding unit 26 is aligned with a respective well 13 when the sample carrier 12 is received by the station 24. Each light guiding unit 26 is configured to direct a beam 28 of excitation radiation to the bottom 15 of the respective well 13, and to direct a beam 30 of reflected radiation from the bottom 15 of the respective well 13. In preferred embodiments, the sample carrier 12 is removably locatable on the station 24. The carrier 12 may be installed manually or by an automated carrier loading device (not illustrated). In alternative embodiments the sample carrier 12 may be fixed in the station 24.

Optionally, one or more a clamp (not shown) may be provided for releasably securing the carrier 12 to the station 24. The clamp(s) may be manually operable or electro-mechanically operably as desired.

Optionally, the reader 14 may comprise an agitation system (not illustrated) for agitating the sample carrier 12 when located in the station 24. The agitation may take any convenient conventional form, for example comprising means for imparting any one or more of acoustic, magnetic or mechanical agitation to the samples within the wells 13.

Optionally, the system 10 includes a temperature control system for controlling the temperature of the samples in the wells 13.

Optionally, the reader 14 includes one or more sensor for detecting the presence of a sample carrier 12 in the station 24.

In preferred embodiments, the light guiding units 26 are arranged in an array corresponding to the array in which the wells 13 are arranged. Each light guiding unit 26 comprises an optical port 32 for optically coupling the light guiding unit 26 to the respective well 13 when the carrier 12 is installed. Each optical port 32 is an optical interface configured to enable the beam 30 of excitation radiation to be directed to from the light guiding unit 26 (in particular from the light guide 31) to the bottom 15 of the respective well 13, and to enable the beam 30 of reflected radiation to be directed from the bottom 15 of the respective well 13 to the light guiding unit 26 (in particular to the light guide 27). The arrangement is such that the respective optical port 32 is aligned with the respective well 13 when the sample carrier 12 located in the station. The station 24 comprises a support structure 34, which is preferably plate-like in form, for receiving the sample carrier 12. In particular, the support structure 34 is shaped and dimensioned to receive the base of the sample carrier 12. The optical ports 32 are included in the support structure 34 and are exposed by the support structure for optically coupling with the respective well 20. The support structure 34 comprises a support surface 36, which is preferably flat or substantially flat, for receiving the base of the sample carrier 12. The optical ports 32 are incorporated into the support surface 36, preferably arranged in an array, and are exposed by the support surface 36 for optically coupling with the respective well 13.

Preferably, each well bottom 15, in particular the reverse face 15B of the well bottom 15, covers the respective optical port 32 when the sample carrier 12 is installed in the station 24. Preferred optical ports 32 have a mouth 33, which is preferably open and which may open onto the surface 36. The arrangement is such that the reverse face 15B of the well bottom 15, covers the mouth 33 of the respective optical port 32 when the sample carrier 12 is seated in the station 24. According to the invention, the base of the sample carrier 12 is seated on the support surface 36 when the carrier 12 is installed in the station 24. Each well 13 may engage with the respective optical port 32, typically with the mouth 33 of the respective optical port 32.

According to the invention, each optical port 32 comprises a cavity 38. The cavity 38 is located below the support surface 36 and opens onto the support surface 36 via the mouth 33. Each light guiding unit 26 is configured to direct the beam 30 of excitation radiation to the bottom 15 of the respective well 13 through the cavity 38, and to direct the beam 30 of reflected radiation from the bottom 15 of the respective well 13 through the cavity 38. Preferably, the cavity 38 is filled with a medium, conveniently air, having a lower refractive index than the material, e.g. glass, from which the bottom 15 of the well 13 is made.

The light guiding units 26 are part of an excitation and detection system for illuminating the nanostructured surface of the well bottoms 15, and for detecting light reflected from, and/or emitted from, the nanostructured surface as a result of the illumination.

In preferred embodiments, each light guiding unit 26 is connected to an excitation source 40 for generating the beam 28 of excitation radiation. The excitation source 40 is conveniently part of the reader device 14, although it may be provided separately from the reader device 14 in which case the light guiding units 26 may be connectable to the excitation source 40 in any conventional manner. Each light guiding unit 26 includes light guiding means, preferably comprising at least one optical fibre 27 (e.g. a single optical fibre or a bundle of optical fibres), for directing the excitation beam 28 to the optical port 32, and so to the bottom 15 of the respective well 13, and in particular to the reverse face 15B, when the sample carrier 12 is installed. In preferred embodiments, the light guiding means is arranged to direct the excitation beam 28 to the cavity 38, whereupon the beam 28 travels through the cavity 38 and the mouth 33, to impinge upon the reverse face 15B of the well bottom 15B when the carrier 12 is installed.

In typical embodiments the excitation source 40 is of a type that generates light, preferably light having a wavelength typically between approximately 300 nm to 1500 nm, typically but not necessarily visible light. In preferred embodiments, the excitation beam 28 comprises polarised light, preferably p-polarised light, also known as transverse-magnetic light. P-polarized light is linearly polarized light with polarization direction lying in the plane of incidence. The plane of incidence is the plane which contains the surface normal and the propagation vector of the incoming light radiation. Polarized light with its electric field along the plane of incidence is thus denoted p-polarized. P polarized radiation is commonly referred to as transverse-magnetic (TM) radiation. The desired polarization may be achieved by any convenient means, for example by providing the excitation source 40 with polarizing filter or, as illustrated, by providing a polarizing filter 29 on the optical fibre 27. The excitation source 40 may comprise one or more light source as is convenient. For example the excitation source 40 may comprise a single light source that provides the excitation beams 28 for all light guiding units 26, or may comprise a respective light source for providing the respective excitation beam 28 for each light guiding unit 26. In preferred embodiments, the, or each, light source comprises an LED, but may take alternative forms, for example an incandescent lamp or a laser device.

In preferred embodiments, each light guiding unit 26 is connected to an optical detector 42 for receiving the beam 30 of reflected radiation. The optical detector 42 is conveniently part of the reader device 14, although it may be provided separately from the reader device 14 in which case the light guiding units 26 may be connectable to the optical detector 42 in any conventional manner.

Each light guiding unit 26 includes light guiding means, preferably comprising at least one optical fibre 31 (e.g. a single optical fibre or a bundle of optical fibres), for directing the reflected beam 30 from the optical port 32 to optical detector 42. In preferred embodiments, the light guiding means is arranged to direct the reflected beam 30 from the cavity 38 to the optical detector 42. The optical detector 42 may comprise one or more light sensing device as is convenient. For example the optical detector 42 may comprise a single light sensor, e,g, a digital image sensor or digital camera, that serves as the optical detector 42 for all of the light guiding units 26, or may comprise a respective light sensor, e.g. a respective photodiode, for each light guiding unit 26 (e.g. the detector 42 may comprise an array of photodiodes or other photosensors). In the case of an image sensor or digital camera, the light guiding means of each light guiding unit 26 may be mapped to one or more pixel of the image sensor.

Each light guiding unit 26 has a body 43 for supporting the light guiding means and in which the cavity 38 is formed. The body 43 comprises an excitation channel 44 for directing the excitation beam 28 to the bottom 15 of the respective well 13. The channel 44 is typically linear and is inclined with respect to the support surface 26 in order to deliver the beam 28 to the reverse face 15B of the well bottom 15 at a desired angle of incidence. The body 43 includes a detection channel 46 for directing the reflected beam 30 to the detector 42. The detection channel 46 is typically linear and is inclined with respect to the support surface 26 in order to receive the reflected beam 30. In preferred embodiments, the channels 44, 46 terminate at the cavity 38. In preferred embodiments, the channels are shaped and dimensioned to hold the respective optical fibre(s) 27, 31.

Optionally, a ball lens or half ball lens 25 is provided at the cavity-end of each fibre 27, 31 for collimating light from or coupling light into, as applicable, the fibre. Optical elements of the system 10 may be fabricated from any suitable material, for example quartz, glass, plastics or sapphire.

In alternative embodiments, the light guiding means may comprise means for focussing and/or collimating one or both of the radiation beams 28, 30, e.g. one or more lenses, and/or the excitation source may be of a type that generates a focussed beam, e.g. a laser, in which case the optical fibres and/or channels may be omitted. Optionally, one or more focussing and/or collimating lenses may be provided at the excitation source and/or at the detector.

In the illustrated embodiment, the body 43 is solid, the channels 44, 46 being formed therein by any conventional means, e.g. drilling or moulding. In alternative embodiments, the body 43 need not be solid. For example it may comprise a frame holding one or more tubes which define the, or each, channel 44, 46, or configured to hold the fibre(s) 27, 31.

In preferred embodiments, the light guiding units 26 are configured so that the excitation beam is incident on the reverse face 15B of the respective well bottom 15 at an angle (measured with respect to the face normal), that is equal to or substantially equal to the Brewster angle (also known as Brewster's angle or the polarisation angle). The Brewster angle is the angle of incidence at which light with a particular polarization, in this case p-polarisation, is perfectly transmitted through the surface, in this case the reverse face 15B, of a transparent medium when passing from a first medium to a second medium which have different refractive indicies, with no reflection from the surface. In this example, the first medium is that of the cavity 38 and is typically air, while the second medium is that of the well bottom 15, which is typically glass. The Brewster angle for an air/glass interface is approximately 57º (where the glass has a refractive index of 1.52). It will be understood that the Brewster angle may be different for different media that may be used in alternative embodiments of the invention, including glass having a different refractive index than 1.52.

Hence, in the preferred embodiment, the channel 44 and/or fibre(s) 27 are angled with respect to the reverse face 15B such that the excitation beam 28 is incident on the reverse face 15B at or substantially at the Brewster angle, thereby eliminating or substantially eliminating reflections from the reverse face 15B.

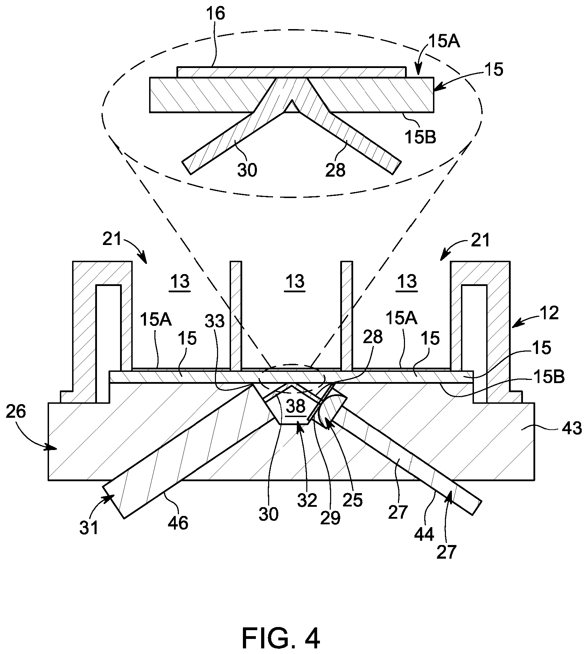

In use, and as can best be seen from the detail of Figure 4, when the excitation beam 28 hits the reverse face 15B, it is refracted by the well bottom 15. Hence, the angle of incidence of the excitation beam 28 at the obverse face 15A of the bottom 15 (with respect to the normal of those surfaces) is less than the angle of incidence of the beam 38 at the reverse face 13A (with respect to the normal). Advantageously, the arrangement is such that the angle of incidence at the obverse face 15A is less than the angle required to achieve attenuated total reflection (ATR) within the bottom 15.

In use, plasmonic oscillations, which may also be referred to as plasmonic resonance, are caused in the nanostructured surface of the obverse face 15A in response to radiation beam 38 incident at the reverse of the nanostructured face 15A of the well bottom 15. Plasmonic oscillations may occur in directions that are possible to be excited by the radiation. In the preferred embodiments where the nanostructures 16 are elongate, plasmonic oscillations occur both along and transverse to the longitudinal axis of the nanostructures 16. The plasmonic oscillations along the longitudinal axis resonances are in this case used for sensing, which requires a component of the excitation light to be at non-normal incidence on the reverse face 15B. In preferred embodiments in which the longitudinal axis of the nanostructures 16 is perpendicular to the face 15A, the plasmonic oscillations occur in a direction perpendicular to the face 15A.

In preferred embodiments, ATR is not possible at face 15A because face 15B is parallel to it. For ATR to occur, a prism (not shown) would have to be used instead of the preferred slide. Accordingly, in preferred embodiments, plasmons can be excited in the nanostructured surface of obverse face 15A with the excitation beam 28 incident at any angle without getting to an ATR angle. The only incidence angle that gives low reflection from parallel face 15B is the Brewster angle.

Advantageously the incident radiation impinges on the reverse face 15B at the Brewster angle to eliminate or substantially eliminate reflections from the face 15B. The incident radiation beam 28 subsequently impinges on the reverse of the nanostructured surface 15B, and is reflected by the nanostructured surface 15B whereupon it travels back through the well bottom 15, emerging from the reverse face 15B and entering the optical port 32 as the reflected radiation beam 30. Advantageously, there is no ATR of the beams 28, 30 within the bottom 15.

The plasmonic oscillations in the nanostructured surface of the well bottom 15 interact with the sample in the well 13 and, depending on what is contained in the sample, these interactions affect one or more characteristics of the reflected beam 30, for example its intensity. For example, changes in the intensity (e.g. a modulation of intensity) can be caused by an alteration of the resonance conditions of the nanostructures 16. Hence, by analysing one or more characteristics of the reflected beam 30, the detector 42 can determine one or more characteristics of the sample, e.g. relating to its composition. Any suitable conventional analysis may be used for this purpose. Optionally, the intensity of the measured light can be used to modify the intensity of the excitation light.

It is noted that Brewster angle is not used to get minimum reflectivity at the reverse face of the nanostructured surface; instead preferred embodiments use the Brewster angle for p-polarised radiation to minimise unwanted reflection from the reverse face 15B of the well bottom 15. This is possible because the nanostructured surface of the obverse face 15A can generate plasmonic oscillations when excited at an angle less than required for ATR. This makes the system simpler and cheaper in comparison with known alternatives that use ATR prisms to create ATR of the light to excite surface plasmons in a gold or silver planar film.

In preferred embodiments, the system 10 includes a controller 48 for controlling the operation of the excitation source 40, in particular to control the illumination of the wells 13 individually or collectively. The controller 48 may be configured to control the excitation source 40 to cause a respective beam of excitation radiation to be directed to the bottom 15 of the respective well 13 by any one of the light guiding units 26 individually, or by any two or more of the light guiding units simultaneously, or by all of the light guiding units simultaneously. The controller 48 may also be configured to gather data from the optical detector 42, advantageously in respect of each well 13 individually.

The controller 48 may take any convenient conventional form, for example comprising a suitably programmed microprocessor or microcontroller or other electronic or data processing means, preferably with one or more data storage device (not shown). Preferably, the controller 48 is included in the reader 14, but may alternatively be provided separately from the reader unit 14, e.g. by an external computer system (not shown) to which the reader 14 is connected, or connectable, by any conventional wired or wireless communication link(s). Accordingly, the reader 14 may include conventional hardware and software for supporting wired or wireless communication.

The preferred system 10 includes analysis means configured to analyze one or more output signal from the optical detector. The analyzing means may be configured to analyze data from one or more output signal in respect of any one of the wells 13 individually, or in respect of any two or more of the wells 13 in combination, or in respect of all of the wells 13 individually or in combination. Conveniently, the analyzing means is implemented by the controller 48. Alternatively, or in addition, the analyzing means may be implemented by any other suitable programmed microprocessor or microcontroller or other electronic or data processing means (not shown), which may be provided on the reader unit 14, or separately from the reader unit 14, e.g. by an external computer system (not shown) to which the reader 14 is connected, or connectable, by any conventional wired or wireless communication link(s).

Analysis of data determined from the output signals may be performed to quantify the amount of biological material in each well 13. In preferred embodiments, the nanostructures 16 cause a uniform plasmonic field to be created, which allows the development of analysing algorithms to quantify the biological material in absolute rather than relative terms e.g. approximate molecules per square millimetre. Quantification analysis may be used to produce a pass/fail system for each well 13. Analysis of the data may be used to determine kinetic information from the biological material in the well 13. One or more quality control check may be performed to calibrate the system 10. Calibration of the system 10 may be performed using a calibration, or control, version of the sample carrier 12 with known optical parameters. The calibration carrier 12 may be patterned such that any mis-alignments in the optical system lead to predictable changes in the detected light.

The reader 14 preferably includes a display device 49, conveniently controlled by the controller 48, for displaying results of the system's analysis and/or information relating to the analysis. The reader 14 may also include a user input device 47, e.g. a key pad and/or touch screen.

To enhance the detection of one or more analytes 55 that may be contained with the samples, the sample may include nanoentities 50 configured to bind with the analytes 55 and/or the nanostructures 16 such that the analytes 55 may be immobilised upon the nanostructured surface when the nanoentities 50 are simultaneously bound to both the analytes 55 and the nanostructures 16.

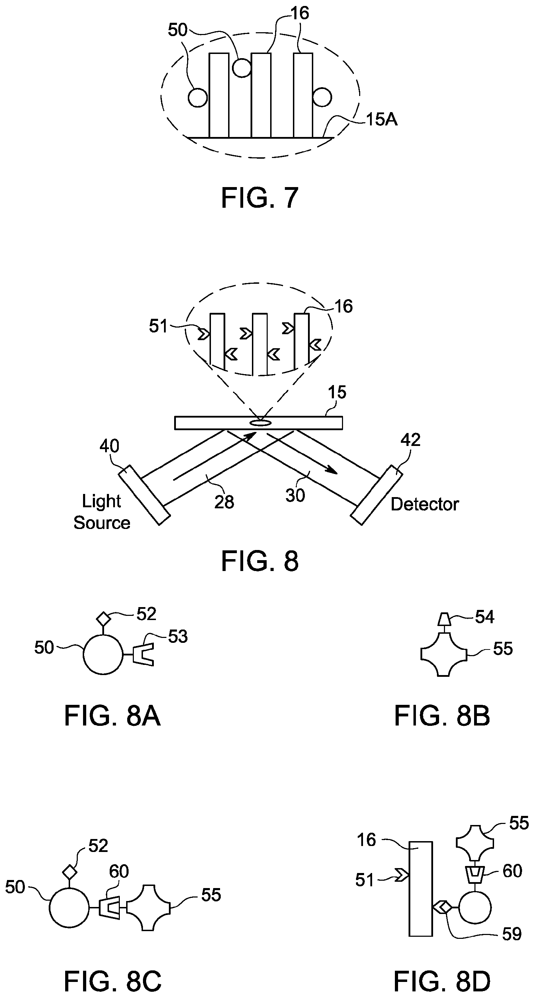

Figure 7 shows a detailed view of the nanostructured surface of the bottom face 15A and in particular shows the plurality of nanoentities 50 located adjacent to the nanostructures 16. The nanoentities 50 may comprise any suitable shape however, preferably, as is shown in the figures the nanoentities may be spherical in shape. The nanoentities 50 are preferably formed from an electrically conductive material, typically a metallic material, for example, any of silver, gold, aluminium, platinum, copper or any noble metal or any combinations of the aforesaid. The nanoentities 50 may be 1nm to 100nm in diameter. The nanoentities 50 are configured to bind to both the nanostructures 16 and the analytes 55 by respective binding couples, wherein each binding couple comprises a first member which has a binding affinity for a second member and vice versa. The first and second members of the binding couples ideally comprise complimentary receptors and ligands such as an antibody and a corresponding antigen such that the binding couple when bound forms an antibody-antigen conjugation. The nanostructures 16 of the nanostructured surface may be functionalised i.e. by coating other any other suitable means including standard biological protocol, with a first member of a binding couple which is operable to bind with a second member of the binding couple located upon the nanoentities 50. Typically the nanoentities 50 may be functionalised i.e. by coating or any other suitable means, with at least an antibody which is co-operable with the antigen of a specific analyte. Advantageously the binding of the nanoentities 50 to the nanostructured surface disrupts the plasmonic resonance excited upon the nanostructured surface resulting in a detectable change in optical reflection of the reflected beam 30 which may be measured by the detector 42 to indicate the presence and/or concentration of one or more analytes 55.

At least part of the nanostructured surface may be coated with a first member 51 of a primary binding couple 59 (as shown in figure 8) having an affinity for a second member 52 of the primary binding couple 59 which is coated upon at least some of the nanoentities 50 (see Figure 8A). Consequently the nanoentities 50 are therefore operable to bind to the nanostructured surface by the binding of the primary binding couple 59. The nanoentities 50 may be further coated with a first member 53 of a secondary binding couple 60 having an affinity for a second member 54 of the secondary binding couple 60 which comprises at least one of the analytes 55 contained within the sample. As a result of the primary and secondary binding couples 59, 60 the analytes 55 contained within the sample may be immobilised upon the nanostructured surface.

The primary and secondary binding couples 59, 60 ideally comprise complimentary receptor and ligands. In a preferred embodiment, as shown in figures 8 to 8D, the nanostructures 16 are coated in the first member 51 of the primary binding couple 59 which comprises a receptor such as an antibody. Referring now to Figure 8A, the nanoentity 50 shown coated in the second member 52 of the primary binding couple 59, which in this case comprises a ligand such as an antigen, which is complementary to the receptor located on the nanostructures 16. Alternatively, the first and second members 51, 52 of the primary binding couple 59 may be reversed such that the ligand is located upon the nanostructures 16 and the receptors are located upon the nanoentities 50. Further as is shown in figure 8A the nanoentity 50 is additionally coated in the first member 53 of the secondary binding couple 60 which in this case comprises a receptor such as an antibody, which is complimentary to the second member 54 of the secondary binding couple 60. A first variation of analyte 55, shown in figure 2B, comprises the second member 54 of the secondary binding couple 60 which typically comprises a ligand, in this case an antigen, which is operable to bind to the receptor, comprising an antibody, located upon the nanoentity 50, as is shown in Figure 8C, to form a nanoentity - analyte complex through conjugation. When the nanoentity 50 is bound to both the analyte 55, the nanostructures 16 by the primary and secondary binding couples 59, 60, the analyte 55 is immobilised on the nanostructured surface as is shown in Figure 8D.

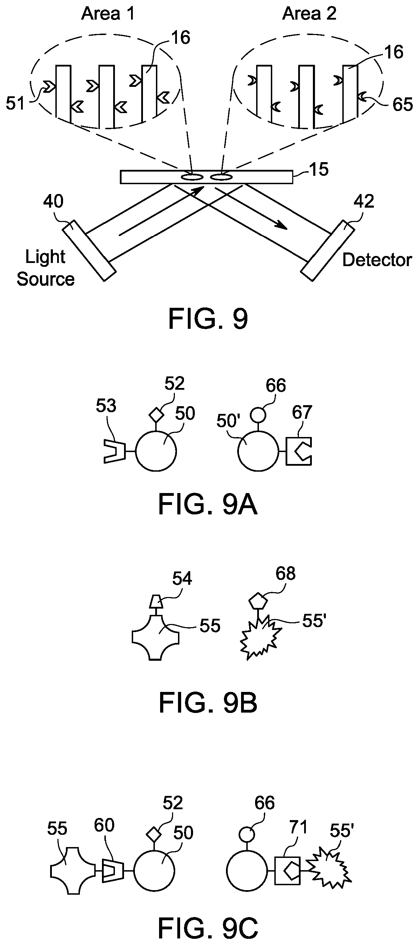

A second region of the nanostructured surface 15A may be coated in a first member 65 of a tertiary binding couple 70 (shown in figure 9) which is operable to bind with a second member 66 of the tertiary binding couple 70 which may be coated upon some of the nanoentities 50' contained within the sample. Further these same nanoentities 50' may be coated in a first member 67 of a quaternary binding couple 71 which has a binding affinity for a second member 68 of the quaternary binding couple 71 which is located upon a second variation of analyte 55'. To this end, as is shown in figures 9 to 9C, the first member 65 of the tertiary binding couple 70 preferably comprises a receptor, in particular an antibody whilst the second member 66 ideally comprises a complimentary ligand, in particular an antigen. Further the first member 67 of the quaternary binding couple 71 typically comprises a receptor, in particular an antibody whilst the second member 68 of the quaternary binding couple 71 preferably comprises a complimentary ligand, in particular an antigen.

**It** should be understood that further regions (not shown) of the nanostructured surface, other nanoentities 50, 50' located within the sample, may be coated in additional binding members (not shown) such that further variations of analytes 55, 55' contained within the sample may be immobilised upon the nanostructured surface of the well bottom 15 in the manner recited above. Additionally one or more regions of the nanostructured surface may be functionalised, typically by coating with at least one binding member which is complimentary to a binding member of one or more **of** the analytes 55, 55' contained within the sample such that the analyte(s) 55, 55' may be directly immobilised upon the nanostructured surface. For example the nanostructured surface may be coated with an antibody which is complimentary with an antigen of a pathogen contained within the sample, such that the pathogen may be immobilised on the nanostructured surface.

The binding between the nanoentities 50, 50' and the nanostructures 16, such as the primary and tertiary binding couples 59, 70, may be effected by a peptide binder or aptamer that has an affinity for the antigen located on the analyte 55. In an alternative embodiment the binding between the first and second members of the secondary binding couple 60 and the first and second members of the tertiary binding couple 70 may be affected by a mixture of antibody/peptide binders and/or aptamers which are operable to interact with a range of antigens on the analytes 55, 55'. Additionally the binding between, the nanoentities 50, 50' and the analytes 55, 55', in particular the binding of the secondary and quaternary binding couples 60, 71, may be effected by a peptide binder or aptamer that has an affinity for the antigen located on the analyte 55. In an alternative embodiment the binding between the first and second members 53, 54 of secondary binding couple 60, or first and second members of the quaternary binding couples may be affected by a mixture of antibody/peptide binders and/or aptamers which are operable to interact with a range of antigens on the analyte 55.

The analyte 55, 55' typically comprises biological material, for example the analyte may comprise a pathogen such as a virus, bacterium or fungus. Alternatively the analyte may comprise a biological element which is secreted or otherwise produced from a cell for example outer membrane vesicles secreted by Gram negative bacteria. Further the analytes 55 may comprise extracellular secreted proteins, human biomarkers, immunoglobulin or human cells.

For each of the embodiments described it is preferable that that the quantity of nanoentities 50, 50' which are mixed with the sample is known prior to their introduction into the sample. Additionally or alternatively, prior to the sample being introduced into the well 13, the sample may undergo separation such that only the particular analytes 55, 55' of which it is desired to measure the concentration of, are introduced into the well with other elements of the sample being removed. In such an embodiment the sample may be mixed with a plurality of nanoentities 50, 50' which are configured to bind with the particular analytes 55, 55'. Preferably, for each embodiment described, upon binding of the nanoentities 50, 50' with the analytes 55, 55' to form binding couples, the sample may then undergo separation to remove the binding couples from the unbound nanoentities 50, 50' and analytes 55, 55'. The binding couples of the nanoentities 50, 50' and the analyte 55, 55' may be separated from the sample by mass or size using a centrifuge or a filter respectively. Subsequently this solution of binding couples may then be introduced into the well 13 for analysis. The analysis may include obtaining measurements of the reflection characteristics of the nanostructured surface when the nanoentity-analyte binding couples are immobilised, by binding, upon the nanostructured surface, these measurements may then be compared with measurements obtained when a known quantity of nanoentities 50, 50' are bound to the nanostructured surface. For example the known quantity may comprise at least one nanoentity 50, 50' bound to the nanostructured surface or more preferably the known quantity may comprise the total quantity of nanoentities 50, 50' which were introduced to the sample prior to separation. By comparing the measurement obtained when the nanoentity-analyte binding couples are bound to the nanostructured surface to the measurement obtained by a known quantity of nanoentities 50, 50' are bound to the nanostructured surface a ratio can be calculated which allows for the concentration of analyte to be determined.

Optionally, at least some of the nanoentities 50, 50' may comprise a fluorescent material for example quantum dots or other semiconductor nanoentities.

Optionally, surface plasmon resonance methodologies may be used to give accurate, easily interpreted information on biological interactions useful in numerous applications for example assay development and screening of new drug candidates. In preferred embodiments, the system 10 may serve as a biological interaction analysis system for recording and interpretation of label free immunoassays through the automated interpretation of plasmonic resonant peak shifts. Advantageously, the system 10 may be configured to monitor plasmon resonance shifts from one or more functionalised zones of an immunoassay and quantify the presence and affinities of analyte presence within a fluid sample. In some embodiments the sample being tested comprises a mixture of nanoentities and analyte. The nanoentities interact with the nanostructures causing a disturbance of the localised surface plasmon resonance conditions. The nanoentities are typically be made from metallic or semiconductor materials and can be functionalised to both pick up particular sample analytes and to bind to various functionalised regions at the bottom 15 of the nanostructured wells 13. The disruption of resonances on binding has an amplification affect that facilitates analysis of the output signals of the detector 42. In the case where the nanoenties comprise fluorescent particles or are functionalised with fluorescent molecules, a simultaneous plasmonic resonance shift and fluorescent signal may be detected in the relevant output signal of the detector 42.

Noble metal nanostructures may be grown in solution and dispersed on the bottom 15 of the wells 13. The nanostructures may be grown from templated processes like porous alumina. Optionally, the sample carriers 12 are made from polymeric or plastics material by nano-imprinting or injection molding and subsequently coated in an ultrathin layer of noble metal. The nanostructures 16 may be coated in a ultra-thin semi-transparent layer of gold (1 -100nm) and thiol chemistry is used to attach biological entities covalently to their surface.

In preferred embodiments the spacing between the nanostructures 16 is less than the wavelength of the light impinging on them in use. In these circumstances neighbouring nanostructures 16 experience significant coupling at the near-field, blue shifting the optical resonance frequency. In the case of gold nanostructures, the resonance peak shifts from the infrared to optical frequencies. This near-field coupling also generates intense electric fields in the vicinity of the nanostructures 16.

In some embodiments, the absolute amount of the biological material attached to the nanostructures 16 can be quantified e.g. molecules per square or cubic millimetre. As the geometry of the nanostructures 16 are well defined, an algorithm can be used to estimate the mass of biological material present.

Advantageously, the optical response of each well 13 can be monitored during biological interaction investigations in real-time. Attaching biomolecules to the nanostructured bottom 15 causes an increase in the local refractive index, redshifting the resonance peak. A broadband light source can be used to measure changes in spectral absorbance with time. Alternatively, an LED of a fixed wavelength overlapping the plasmon resonance peak can be used to measure changes in intensity or the transmitted or reflected light with time.

In some embodiments, the system 10 may be configured to measure the kinetic profile of protein-protein interactions in at least one well 13. In this case the nanostructured bottom 15A is coated in a specific receptor to an analyte of interest. A solution containing the analyte is introduced into the well 13 and a kinetic curve is generated as the analyte binds to the receptor. From the kinetic curve, association and dissociation curves, and affinity constants, can be generated.

In some embodiments, the system 10 may be configured to monitor and optimise assay development. Standard immunoassays are developed in a 96-well plate format, wherein the capture antibody/protein is first immobilised to one or more wells and blocked with a smaller protein (e.g. casein or BSA). The analyte containing the pathogen of interest may be introduced into the well 13, and if present binds to the receptor. A secondary antibody conjugated with an enzyme (e.g. TMB) is used to read the concentration of the pathogen colorimetrically. The conventional assay development process contains multiple steps (immobilisation, blocking, detection, amplification, development) that can fail at any stage. The end user only gets an end-point readout which may not be truly indicative of pathogen detection. Some embodiments of the invention may be used to monitor attachments to the bottom 15 of at least one of the nanostructured wells 13 during each stage of the assay development process, wherein kinetic curves are generated and an algorithm is used to determine whether each stage has passed.

In some embodiments the may be configured to screen phage display libraries. The genetic encoding of bacteriophages are varied to create a range of surface proteins which are screened against a target antibody. For example, the system 10 may be used in a single, 8, 24, 48, 96, 192, 384 or 1536 well plate format to analyse the binding efficiency of the phage library to an antibody immobilised to the nanostructured base. Kinetic curves are generated showing association and dissociation rates and an algorithm can be used to rank the phage library, accordingly.

Advantageously, the nanostructured sample carriers 12 may be used to improve the performance of fluorescent immunoassays through a phenomenon known as plasmon enhanced fluorescence. The localised electric fields from the plasmon resonance can cause the emission from a fluorophore, in close proximity to the nanostructures 16, to be amplified by up to 100 fold. The system 10 can be used to enhance the limits of detection of fluorescently tagged biomolecules in one or more wells 13.

In some embodiments, the nanostructured sample carrier 12 facilitates measuring the presence of molecules attached to the surface of the nanostructures 16 using surface enhanced Raman spectroscopy (SERS). SERS is a surface technique that uses plasmonics resonances and their intense localised electric fields to enhance Raman scattering by factors of up to 10¹¹ and can be used for single molecule detection. The spectrum of the Raman-scattered light depends on the molecular constituents present, wherein each biomolecule or protein will have its own Raman fingerprint. Optionally, the system 10 may generate Raman spectra to identify and quantify the constituents of an analyte solution.

In some embodiments, at least one nanostructured well 13 may be used as a "lab in a well". The plasmon resonance shift through changes in mass may be utilised to monitor the presence of cells and once captured to the nanostructured bottom 15, their subsequent growth and / or death in different ambient conditions. Concurrently, cell surface expressions (e.g. glycans) can be monitored using their surface enhanced Raman spectroscopy fingerprint or using fluorescently tagged binders. In some embodiments, at least one nanostructured well 13 may be used as a second "lab in a well". The shape and structure of cells in the wells 13 can be imaged using fluorescent dye, a confocal microscope set-up and a camera-based detection system. Concurrently, inflammatory biomarker levels (e.g. cytokines) can be monitored by tracking plasmon resonance shift as the cells are exposed to different pathogens or therapeutics. The sample carrier 12 in this case may be spotted with an array of binders for the biomarkers of interest and thus multiplexed analysis can be achieved. Further to this, any changes in the surface proteins expressed by the cells can be monitored concurrently using surface enhanced Raman spectroscopy.

The invention is not limited to the embodiment(s) described herein but can be amended or modified without departing from the scope of the present invention, which is limited solely by the appended claims.

## Claims

1. A reader device (14) for an analyzing system the reader device (14) comprising a station (24) for receiving a multi-well sample carrier (12), the station comprising a support structure (34) that is shaped and dimensioned to receive a base of said sample carrier (12), said support structure having a support surface (36) for seating said base of said sample carrier (12), the station (24) comprising a plurality of light guiding units (26) arranged so that a respective light guiding unit (26) is aligned with a respective well (13) of the sample carrier (12) when the sample carrier (12) is received by the station (24), wherein each light guiding unit (26) comprises an optical port (32) for optically coupling the light guiding unit (26) with the respective well (13) when the sample carrier (12) is received by the station (24), each optical port (32) being included in said support structure (34) and being exposed by the support surface (36) for coupling with the respective well (13), and wherein each optical port (32) comprises a cavity (38), located below said support surface (36) and opening onto said support surface (36) via a mouth (33), the arrangement being such that a bottom of the respective well (13) covers the mouth (33) when the base of the sample carrier (12) is seated on the support surface (36), and wherein each light guiding unit (26) is connected to, or connectable to, an excitation source (40) for generating a beam of excitation radiation (28), each light guiding unit comprising excitation light guiding means for directing said beam of excitation radiation to the bottom of the respective well (13) through the respective cavity (38), and reflected light guiding means for directing a beam of reflected radiation (30) from the bottom of the respective well (13) through the respective cavity (38), and wherein each light guiding unit (26) comprises a body (43) in which said cavity (38) is formed, said body (43) comprising an excitation channel (44) for said excitation light guiding means, said excitation channel (44) being inclined with respect to the support surface (36) in order to deliver the beam of excitation light to a reverse face of the well bottom at a desired angle of incidence, the body including a detection channel (46) for said reflected light guiding means, said detection channel (46) being inclined with respect to the support surface (36) in order to receive said beam of reflected radiation.

2. An analyzing system comprising:
the multi-well sample carrier (12) comprising a plurality of sample wells (13), each well having a transparent bottom (15), the bottom having an obverse face (15A) located within the well (13) and a reverse face (15B) external to the well (13), the obverse face (15A) having a nanostructured surface comprising a plurality of nanostructures (16); and
the reader device (14) according to claim 1.

3. The system of claim 2, wherein the sample carrier (12) is removably locatable on the station (24).

4. The system of claim 2 or 3, wherein said wells (13) are arranged in an array, and said light guiding units (26) are arranged in an array corresponding to the array in which the wells (13) are arranged.

5. The system of any of claim 2-4, wherein the respective well bottoms, in particular the respective reverse faces (15B) of the well bottoms, are located at said base and preferably are coplanar, or substantially coplanar, with one another.

6. The system of any of claims 2-5, wherein the respective well (13) engages with the respective mouth (33) of the respective cavity (38) when the sample carrier (12) is received by the station (24).

7. The system of any of claims 2-6, wherein said light guiding means (26) comprises means for focusing and/or collimating the respective radiation beam.

8. The system of any of claims 2-7, wherein said light guiding means (26) comprises at least one optical fibre.

9. The system of any of claims 2-8, wherein each light guiding unit (26) is connected to, or connectable to, an optical detector (42), said light guiding means (26) being arranged to direct said reflected beam light from the bottom of the respective well (13) through said cavity (38) to said detector (42).

10. The system of any of claims 2-9, wherein said nanostructures (16) are elongate, having a respective longitudinal axis that is disposed substantially perpendicularly to the obverse face (15A), and wherein, preferably, the nanostructures (16) are spaced apart from one another by a distance less than the wavelength of the excitation radiation to cause, in use, plasmonic oscillations in a direction that is normal to said obverse face.

11. The system of any of claims 2-10 further comprising: an excitation source (40) for generating the beam of excitation radiation for each light guiding unit (26); an optical detector (42) for detecting the beam of reflected radiation from each light guiding unit (26); and a controller (48) for controlling operation of the excitation source(40); wherein the controller (48) is configured to control the excitation source (40) to cause a respective beam of excitation radiation to be directed to the bottom of the respective well (13) by any one of the light guiding units (26) individually, or by any two or more of the light guiding units (26) simultaneously, or by all of the light guiding units (26) simultaneously.

12. The system of claim 11, further including analyzing means configured to analyze one or more output signal from said optical detector (42), wherein said analyzing means is configured to analyze data from said at least one output signal in respect of any one of the wells individually, or in respect of any two or more of the wells in combination, or in respect of all of the wells individually or in combination.

13. The system of any of claims 2-12, wherein at least a first region of the nanostructured surface is functionalised with a first member of a primary binding couple having an affinity for a second member of the primary binding couple which is functionalised upon at least some of the nanoentities; and/or wherein the nanoentities are further functionalised with a first member of a secondary binding couple having an affinity for a second member of the secondary binding couple which comprises at least one of the analytes contained within the sample; and/or wherein the analyte is functionalised with the second member of the secondary binding couple.

14. The system of any of claims 2-13, wherein the light guiding units (26) are configured so that the excitation beam is incident on the reverse face (15B) of the respective well bottom at an angle that is equal to or substantially equal to the Brewster angle.

## Patentansprüche

1. Lesevorrichtung (14) für ein Analysesystem, wobei die Lesevorrichtung (14) eine Station (24) zum Aufnehmen eines Probenträgers (12) mit mehreren Vertiefungen umfasst, wobei die Station eine Stützstruktur (34) umfasst, die geformt und dimensioniert ist, um eine Basis des Probenträgers (12) aufzunehmen, wobei die Stützstruktur eine Stützoberfläche (36) zum Aufsetzen der Basis des Probenträgers (12) aufweist, wobei die Station (24) eine Vielzahl von Lichtleiteinheiten (26) umfasst, die so angeordnet sind, dass eine jeweilige Lichtleiteinheit (26) mit einer jeweiligen Vertiefung (13) des Probenträgers (12) ausgerichtet ist, wenn der Probenträger (12) durch die Station (24) aufgenommen wird, wobei jede Lichtleiteinheit (26) eine optische Öffnung (32) zum optischen Koppeln der Lichtleiteinheit (26) mit der jeweiligen Vertiefung (13) umfasst, wenn der Probenträger (12) durch die Station (24) aufgenommen wird, wobei jede optische Öffnung (32) in der Stützstruktur (34) beinhaltet ist und durch die Stützoberfläche (36) zum Koppeln mit der jeweiligen Vertiefung (13) freigelegt ist, und wobei jede optische Öffnung (32) einen Hohlraum (38) umfasst, der unter der Stützoberfläche (36) verortet ist und sich über eine Mündung (33) auf die Stützoberfläche (36) öffnet, wobei die Anordnung derart ist, dass ein Boden der jeweiligen Vertiefung (13) die Mündung (33) abdeckt, wenn die Basis des Probenträgers (12) auf der Stützoberfläche (36) aufsitzt, und wobei jede Lichtleiteinheit (26) mit einer Anregungsquelle (40) zum Erzeugen eines Strahls von Anregungsstrahlung (28) verbunden oder verbindbar ist, wobei jede Lichtleiteinheit ein Anregungslichtleitmittel zum Richten des Strahls der Anregungsstrahlung durch den jeweiligen Hohlraum (38) auf den Boden der jeweiligen Vertiefung (13) und ein Reflexionslichtleitmittel zum Richten eines Strahls von reflektierter Strahlung (30) von dem Boden der jeweiligen Vertiefung (13) durch den jeweiligen Hohlraum (38) umfasst und wobei jede Lichtleiteinheit (26) einen Körper (43) umfasst, in dem der Hohlraum (38) ausgebildet ist, wobei der Körper (43) einen Anregungskanal (44) für das Anregungslichtleitmittel umfasst, wobei der Anregungskanal (44) in Bezug auf die Stützoberfläche (36) geneigt ist, um den Strahl von Anregungslicht zu einer Rückfläche des Vertiefungsbodens bei einem gewünschten Einfallswinkel zu liefern, wobei der Körper einen Detektionskanal (46) für das Reflexionslichtleitmittel beinhaltet, wobei der Detektionskanal (46) in Bezug auf die Stützoberfläche (36) geneigt ist, um den Strahl der reflektierten Strahlung aufzunehmen.

2. Analysesystem, umfassend:
den Probenträger (12) mit mehreren Vertiefungen, umfassend eine Vielzahl von Probenvertiefungen (13), wobei jede Vertiefung einen transparenten Boden (15) aufweist, wobei der Boden eine innerhalb der Vertiefung (13) verortete Vorderseite (15A) und eine außerhalb der Vertiefung (13) verortete Rückseite (15B) aufweist, wobei die Vorderseite (15A) eine nanostrukturierte Oberfläche, umfassend eine Vielzahl von Nanostrukturen (16), aufweist; und
die Lesevorrichtung (14) nach Anspruch 1.

3. System nach Anspruch 2, wobei der Probenträger (12) abnehmbar auf der Station (24) verortbar ist.

4. System nach Anspruch 2 oder 3, wobei die Vertiefungen (13) in einem Array angeordnet sind und die Lichtleiteinheiten (26) in einem Array angeordnet sind, das dem Array entspricht, in dem die Vertiefungen (13) angeordnet sind.

5. System nach einem der Ansprüche 2 bis 4, wobei die jeweiligen Vertiefungsböden, insbesondere die jeweiligen Rückseiten (15B) der Vertiefungsböden, an der Basis verortet sind und vorzugsweise koplanar oder im Wesentlichen koplanar zueinander sind.

6. System nach einem der Ansprüche 2 bis 5, wobei die jeweilige Vertiefung (13) in die jeweilige Mündung (33) des jeweiligen Hohlraums (38) eingreift, wenn der Probenträger (12) durch die Station (24) aufgenommen wird.

7. System nach einem der Ansprüche 2 bis 6, wobei das Lichtleitmittel (26) ein Mittel zum Fokussieren und/oder Kollimieren des jeweiligen Strahlungsstrahls umfasst.

8. System nach einem der Ansprüche 2 bis 7, wobei das Lichtleitmittel (26) mindestens eine optische Faser umfasst.

9. System nach einem der Ansprüche 2 bis 8, wobei jede Lichtleiteinheit (26) mit einem optischen Detektor (42) verbunden oder verbindbar ist, wobei das Lichtleitmittel (26) angeordnet ist, um das reflektierte Strahlenlicht von dem Boden der jeweiligen Vertiefung (13) durch den Hohlraum (38) auf den Detektor (42) zu richten.

10. System nach einem der Ansprüche 2 bis 9, wobei die Nanostrukturen (16) länglich sind, eine jeweilige Längsachse aufweisen, die im Wesentlichen perpendikulär zu der Vorderseite (15A) positioniert ist, und wobei die Nanostrukturen (16) vorzugsweise durch einen Abstand voneinander beabstandet sind, der geringer als die Wellenlänge der Anregungsstrahlung ist, um bei Verwendung plasmonische Schwingungen in einer Richtung zu bewirken, die senkrecht zu der Vorderseite ist.

11. System nach einem der Ansprüche 2 bis 10, ferner umfassend:
eine Anregungsquelle (40) zum Erzeugen des Strahls der Anregungsstrahlung für jede Lichtleiteinheit (26); einen optischen Detektor (42) zum Detektieren des Strahls der reflektierten Strahlung von jeder Lichtleiteinheit (26); und
eine Steuerung (48) zum Steuern eines Betriebs der Anregungsquelle (40); wobei die Steuerung (48) dazu konfiguriert ist, die Anregungsquelle (40) zu steuern, um zu bewirken, dass ein jeweiliger Strahl der Anregungsstrahlung durch eine beliebige der Lichtleiteinheiten (26) einzeln oder durch beliebige zwei oder mehr der Lichtleiteinheiten (26) gleichzeitig oder durch alle der Lichtleiteinheiten (26) gleichzeitig auf den Boden der jeweiligen Vertiefung (13) gerichtet wird.

12. System nach Anspruch 11, das ferner ein Analysemittel beinhaltet, das dazu konfiguriert ist, ein oder mehrere Ausgangssignale von dem optischen Detektor (42) zu analysieren, wobei das Analysemittel dazu konfiguriert ist, Daten von dem mindestens einen Ausgangssignal in Bezug auf eine beliebige der Vertiefungen einzeln oder in Bezug auf beliebige zwei oder mehr der Vertiefungen in Kombination oder in Bezug auf alle Vertiefungen einzeln oder in Kombination zu analysieren.

13. System nach einem der Ansprüche 2 bis 12, wobei mindestens ein erster Bereich der nanostrukturierten Oberfläche mit einem ersten Element eines primären Bindungspaares funktionalisiert ist, das eine Affinität für ein zweites Element des primären Bindungspaares aufweist, das auf mindestens einigen der Nanoteilchen funktionalisiert ist; und/oder wobei die Nanoteilchen ferner mit einem ersten Element eines sekundären Bindungspaares funktionalisiert sind, das eine Affinität für ein zweites Element des sekundären Bindungspaares aufweist, das mindestens einen der innerhalb der Probe enthaltenen Analyten umfasst; und/oder wobei der Analyt mit dem zweiten Element des sekundären Bindungspaares funktionalisiert ist.

14. System nach einem der Ansprüche 2 bis 13, wobei die Lichtleiteinheiten (26) so konfiguriert sind, dass der Anregungsstrahl auf die Rückseite (15B) des jeweiligen Vertiefungsbodens in einem Winkel auftrifft, der gleich oder im Wesentlichen gleich dem Brewster-Winkel ist.

## Revendications

1. Dispositif de lecture (14) pour un système d'analyse, le dispositif de lecture (14) comprenant une station (24) pour recevoir un support d'échantillons à puits multiples (12), la station comprenant une structure de support (34) qui est formée et dimensionnée pour recevoir une base dudit support d'échantillons (12), ladite structure de support ayant une surface de support (36) pour accueillir ladite base dudit support d'échantillons (12), la station (24) comprenant une pluralité d'unités de guidage de lumière (26) agencées de sorte qu'une unité de guidage de lumière (26) respective est alignée avec un puits (13) respectif du support d'échantillons (12) lorsque le support d'échantillons (12) est reçu par la station (24), dans lequel chaque unité de guidage de lumière (26) comprend un port optique (32) pour coupler optiquement l'unité de guidage de lumière (26) avec le puits (13) respectif lorsque le support d'échantillons (12) est reçu par la station (24), chaque port optique (32) étant inclus dans ladite structure de support (34) et étant exposé par la surface de support (36) pour le couplage avec le puits (13) respectif, et dans lequel chaque port optique (32) comprend une cavité (38), située sous ladite surface de support (36) et s'ouvrant sur ladite surface de support (36) via un orifice (33), l'agencement étant de sorte qu'un fond du puits (13) respectif recouvre l'orifice (33) lorsque la base du support d'échantillons (12) repose sur la surface de support (36), et dans lequel chaque unité de guidage de lumière (26) est connectée à, ou peut être connectée à, une source d'excitation (40) pour générer un faisceau de rayonnement d'excitation (28), chaque unité de guidage de lumière comprenant un moyen de guidage de lumière d'excitation pour diriger ledit faisceau de rayonnement d'excitation vers le fond du puits (13) respectif à travers la cavité (38) respective, et un moyen de guidage de lumière réfléchie pour diriger un faisceau de rayonnement réfléchi (30) à partir du fond du puits (13) respectif à travers la cavité (38) respective, et dans lequel chaque unité de guidage de lumière (26) comprend un corps (43) dans lequel ladite cavité (38) est formée, ledit corps (43) comprenant un canal d'excitation (44) pour ledit moyen de guidage de lumière d'excitation, ledit canal d'excitation (44) étant incliné par rapport à la surface de support (36) afin de délivrer le faisceau de lumière d'excitation à une face arrière du fond de puits selon un angle d'incidence souhaité, le corps comportant un canal de détection (46) pour ledit moyen de guidage de lumière réfléchie, ledit canal de détection (46) étant incliné par rapport à la surface de support (36) afin de recevoir ledit faisceau de rayonnement réfléchi.

2. Système d'analyse comprenant :
le support d'échantillons à puits multiples (12) comprenant une pluralité de puits d'échantillons (13), chaque puits ayant un fond transparent (15), le fond ayant une face avant (15A) située à l'intérieur du puits (13) et une face arrière (15B) extérieure au puits (13), la face avant (15A) ayant une surface nanostructurée comprenant une pluralité de nanostructures (16) ; et
le dispositif de lecture (14) selon la revendication 1.

3. Système selon la revendication 2, dans lequel le support d'échantillons (12) est amovible et positionnable sur la station (24).

4. Système selon la revendication 2 ou 3, dans lequel lesdits puits (13) sont agencés en un réseau, et lesdites unités de guidage de lumière (26) sont agencées en un réseau correspondant au réseau dans lequel les puits (13) sont agencés.

5. Système selon l'une quelconque des revendications 2 à 4, dans lequel les fonds de puits respectifs, en particulier les faces arrière respectives (15B) des fonds de puits, sont situés au niveau de ladite base et sont de préférence coplanaires, ou sensiblement coplanaires, les uns par rapport aux autres.

6. Système selon l'une quelconque des revendications 2 à 5, dans lequel le puits (13) respectif vient en prise avec l'orifice (33) respectif de la cavité (38) respective lorsque le support d'échantillons (12) est reçu par la station (24).

7. Système selon l'une quelconque des revendications 2 à 6, dans lequel ledit moyen de guidage de lumière (26) comprend un moyen de focalisation et/ou de collimation du faisceau de rayonnement respectif.

8. Système selon l'une quelconque des revendications 2 à 7, dans lequel ledit moyen de guidage de lumière (26) comprend au moins une fibre optique.

9. Système selon l'une quelconque des revendications 2 à 8, dans lequel chaque unité de guidage de lumière (26) est connectée à, ou peut être connectée à, un détecteur optique (42), ledit moyen de guidage de lumière (26) étant agencé pour diriger ledit faisceau lumineux réfléchi à partir du fond du puits (13) respectif à travers ladite cavité (38) vers ledit détecteur (42).

10. Système selon l'une quelconque des revendications 2 à 9, dans lequel lesdites nanostructures (16) sont allongées, ayant un axe longitudinal respectif qui est disposé sensiblement perpendiculairement à la face avant (15A), et dans lequel, de préférence, les nanostructures (16) sont espacées les unes des autres d'une distance inférieure à la longueur d'onde du rayonnement d'excitation pour entraîner, en utilisation, des oscillations plasmoniques dans une direction normale à ladite face avant.

11. Système selon l'une quelconque des revendications 2 à 10 comprenant en outre : une source d'excitation (40) pour générer le faisceau de rayonnement d'excitation pour chaque unité de guidage de lumière (26) ; un détecteur optique (42) pour détecter le faisceau de rayonnement réfléchi à partir de chaque unité de guidage de lumière (26) ; et un dispositif de commande (48) pour commander le fonctionnement de la source d'excitation (40) ; dans lequel le dispositif de commande (48) est configuré pour commander la source d'excitation (40) pour amener un faisceau de rayonnement d'excitation respectif à être dirigé vers le fond du puits (13) respectif par l'une quelconque des unités de guidage de lumière (26) individuellement, ou par deux quelconques des unités de guidage de lumière (26) ou plus simultanément, ou par toutes les unités de guidage de lumière (26) simultanément.

12. Système selon la revendication 11, comportant en outre un moyen d'analyse configuré pour analyser un ou plusieurs signaux de sortie provenant dudit détecteur optique (42), dans lequel ledit moyen d'analyse est configuré pour analyser des données provenant dudit au moins un signal de sortie concernant l'un quelconque des puits individuellement, ou concernant deux quelconques ou plus des puits en combinaison, ou concernant tous les puits individuellement ou en combinaison.

13. Système selon l'une quelconque des revendications 2 à 12, dans lequel au moins une première région de la surface nanostructurée est fonctionnalisée avec un premier élément d'un couple de liaison primaire ayant une affinité pour un second élément du couple de liaison primaire qui est fonctionnalisé sur au moins certaines des nanoentités ; et/ou dans lequel les nanoentités sont en outre fonctionnalisées avec un premier élément d'un couple de liaison secondaire ayant une affinité pour un second élément du couple de liaison secondaire qui comprend au moins un des analytes contenus à l'intérieur de l'échantillon ; et/ou dans lequel l'analyte est fonctionnalisé avec le second élément du couple de liaison secondaire.

14. Système selon l'une quelconque des revendications 2 à 13, dans lequel les unités de guidage de lumière (26) sont configurées de sorte que le faisceau d'excitation est incident sur la face arrière (15B) du fond de puits respectif selon un angle qui est égal ou sensiblement égal à l'angle de Brewster.
